# EUROPEAN PATENT APPLICATION

(11) **EP 4 674 473 A1**
(43) Date of publication of application: **07.01.2026**
(21) Application number: 24315328.5
(22) Date of filing: 04.07.2024
(51) Int. Cl.: A61N 1/365, A61N 1/375, A61N 1/05, A61B 5/349, A61B 5/11

(54) **ENERGY-EFFICIENT ACTIVITY-DEPENDENT PACING**

(71) Applicant: Sorin CRM SAS, 92140 Clamart (FR)
(72) Inventor: Beguin, Emmanuel, 78180 Montigny-le-Bretonneux (FR); Vitali, Luca Guido, 10019 Strambino (TO) (IT); Matovic, Helena, 92130 Issy-les-Moulineaux (FR)
(74) Representative: Page, White & Farrer Germany LLP

(57) **Abstract**

This invention relates to a medical device and a method for controlling a heart rate of a patient, wherein a sensor is used for detecting an acceleration of the medical device in two or more different directions and a controller is configured to activate the sensor with different timing in a rest mode and in an active mode to sample the detected acceleration. In the rest mode, the detected acceleration is sampled in the two or more different directions a predetermined number of times per cardiac cycle during a calm period of the cardiac cycle with low signal amplitude, and in the active mode, the detected acceleration is sampled per one or more cardiac cycles in one direction only and during a specific adaptive window that includes a signal of interest, e.g., a systolic component of an endocardial acceleration of the cardiac cycle.

## Description

### FIELD OF THE INVENTION

The invention relates to activity-dependent (e.g., rate responsive) pacing systems for controlling the heart rate of a patient.

### BACKGROUND OF THE INVENTION

The heart is a complex organ precisely controlled by the interplay of electrical and mechanical functions. It consists of four chambers (left and right atria (LA, RA) and left and right ventricles (LV, RV)) connected by four valves, which act in concert to regulate its filling, ejection, and overall pump function.

Pacing systems (such as pacemakers, defibrillators etc.) are used to treat patients with slow heart rate or symptomatic heart blocks or heart failures. They are generally composed of a pulse generator that generates an electrical current required for stimulation of heart musculature and one or two electrodes (also referred to as leads), which are responsible for transmitting the electrical activity generated by the pulse generator to the heart musculature.

Rate-responsive (adaptive) pacing is a form of activity-dependent pacing and employs sensor(s) to detect physical or physiological indices and mimic the response of the normal sinus node. Modern cardiac pacemakers are equipped with a function that allows the heart rate to adapt to the current needs of the patient in situations of increased demand related to exercise and stress (rate response function). This function may be based on a variety of mechanisms, such as a built-in accelerometer responding to increased chest movement or algorithms sensing metabolic demand for oxygen, analysis of intrathoracic impedance, and analysis of the heart rhythm (Q-T interval). The latest technologies in the field of rate-responsive pacing relate to the use of an accelerometer in leadless endocavitary pacemakers.

However, in case of leadless pacemakers, a new approach is needed because the conditions to exploit properly the sensor signal are much more challenging compared to the traditional methods of implementation of the rate response function based on a detection of the chest movements. The orientation of the sensor is not known. The heart sound interferes with the acceleration generated by the movement of the patient. The battery capacity is limited, so strategies to reduce the power consumption are needed, as any power saving enhances device longevity, which is a highly desired benefit.

### SUMMARY OF THE INVENTION

It is an object of the present invention to provide a pacing method and device with improved adaptation to patient's activity and/or reduced energy consumption.

This object is achieved by an implantable medical device as claimed in claim 1, a method as claimed in claim 12, and a computer program product as claimed in claim 15.

According to first aspect, a medical device for controlling a heart rate of a patient is provided, wherein the medical device comprises:
a sensor for detecting an acceleration of the medical device in two or more different directions;
a controller for activating the sensor with different timings in a rest mode and in an active mode to sample the detected acceleration;
wherein the controller is configured to:
   - in the rest mode, sample the detected acceleration in the two or more different directions a predetermined number of times per cardiac cycle during a calm period of the cardiac cycle with low signal amplitude; and
   - in the active mode, sample the detected acceleration per one or more cardiac cycles in one direction only and during a specific adaptive window that includes a signal of interest, in particular a systolic component of an endocardial acceleration of the cardiac cycle.

According to a second aspect, a method of controlling a medical device that controls a heart rate of a patient is provided, wherein the method comprises:
detecting an acceleration of the medical device in two or more different directions;
providing a rest mode in which the detected acceleration is sampled in the two or more different directions a predetermined number of times per one or more cardiac cycles during a calm period of the cardiac cycle with low signal amplitude; and
providing an active mode in which the detected acceleration is sampled per one or more cardiac cycles in one direction only and during a specific adaptive window that includes a signal of interest, in particular a systolic component of an endocardial acceleration of the cardiac cycle.

According to a third aspect, a computer program product is provided, which comprises code means for producing the steps of the above method of the second aspect when run on a computer device.

Accordingly, energy consumption can be reduced, as the sensor is activated less frequently during a calm period of the cardiac cycle only. There are long periods of rest during the day, when the patient is sleeping or sitting and thus not active. The resultant lower power consumption can be further reduced in the active mode by activating the sensor to detect the signal of interest during a specific and adaptive window only. Moreover, the proposed selected use of one or more sensing directions allows using heart sound as input.

According to a first option of any one of the first to third aspects, the medical device may be an implantable medical device, in particular a leadless pacemaker.

According to a second option which may be combined with the first option or any one of the first to third aspects, the detected acceleration may be sampled (e.g., by the controller) in three different directions in the rest mode.

According to a third option which may be combined with any one of the first and second options or any one of the first to third aspects, the detected acceleration may be sampled (e.g., by the controller) at least once per one or more cardiac cycles in the rest mode.

According to a fourth option which may be combined with any one of the first to third options or any one of the first to third aspects, a movement of the patient may be detected (e.g., by the controller) when a variation of a sampled value of the detected acceleration exceeds a predefined threshold on at least one of the two or more different directions in the rest mode.

According to a fifth option which may be combined with any one of the first to fourth options or any one of the first to third aspects, a movement of the patient may be detected (e.g., by the controller) when a predefined threshold is exceeded on at least one of the two or more different directions, e.g., on one cardiac cycle only or on consecutive measurements which may be performed on consecutive cardiac cycles.

According to a sixth option which may be combined with any one of the first to fifth options or any one of the first to third aspects, an enlarged window may be used (e.g., by the controller) for intermediate calibration of the window position at the beginning of the active mode and/or periodically during an acceleration measurement in the active mode.

According to a seventh option which may be combined with anyone of the first to sixth options or any one of the first to third aspects, the rest mode and the active mode may be used (e.g., by the controller) for rate responsive pacing.

According to an eighth option which may be combined with any one of the first to seventh options or any one of the first to third aspects, wherein the heart rate of the patient may be controlled (e.g., by the medical device) in the active mode by following a predetermined relation between an endocardial acceleration amplitude and a pacing rate.

According to a ninth option which may be combined with any one of the first to eighth options or any one of the first to third aspects, a switch from the active mode to the rest mode may be performed (e.g., by the controller) when no movement of the patient is detected for a predetermined number of samples and/or when the endocardial acceleration has decreased to a predetermined level.

According to a tenth option which may be combined with any one of the first to ninth options or any one of the first to third aspects, wherein the pacing rate of the patient may be decreased (e.g., by the controller) after a switch from the active mode to the rest mode by using a predetermined slope.

It is noted that the above device may be implemented based on discrete hardware circuitries with discrete analog and/or digital hardware components, integrated chips, or arrangements of chip modules, or based on signal processing devices or chips controlled by software routines or programs stored in memories, written on a computer readable media, or downloaded from a network, such as the Internet.

It shall be understood that the device of claim 1, the method of claim 12, and the computer program product of claim 15 may have similar and/or identical preferred embodiments, in particular, as defined in the dependent claims.

It shall be understood that a preferred embodiment of the invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

In the following drawings:
Fig. 1 shows schematically a block diagram of a rate-responsive pacing system according to various embodiments;
Fig. 2 shows a flow diagram of a mode switching procedure for a rate-responsive pacing, according to various embodiments;
Fig. 3 shows schematically a state diagram of a rate-responsive pacing system; and
Fig. 4 shows respective waveforms of electrograms (EGMs) and sensor signals in different modes of the rate-response pacing system according to an embodiment.

### DETAILED DESCRIPTION OF EMBODIMENTS

Various embodiments of the present invention are now described based on a rate responsive pacing system.

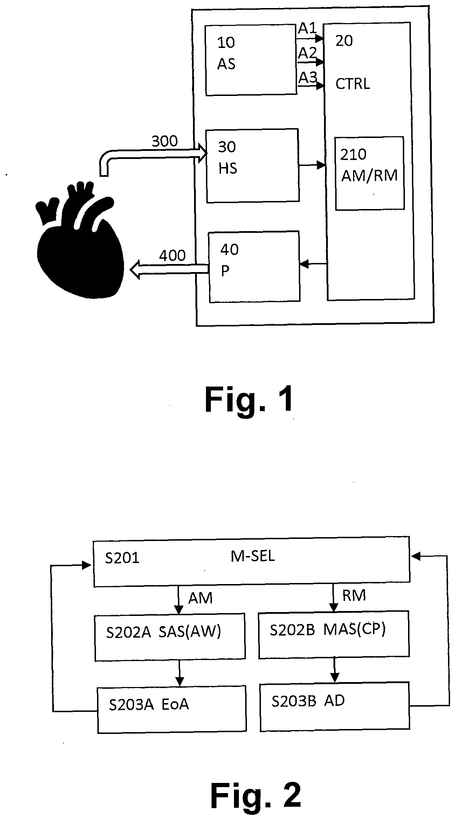

Fig. 1 shows schematically a block diagram of a rate-responsive pacing system according to various embodiments.

It is noted that - throughout the present disclosure - only those structural elements and functions are shown and/or described, which are useful to understand the embodiments. Other structural elements and functions are omitted for brevity reasons.

The pacing system of Fig. 1 can be implemented as an integrated (centralized) or distributed (decentralized) system comprising at least one controller (CTRL) 20 for determining and/or tracking a heart function based on a heart sensing device or system (HS) 30, which may comprise at least one sensing electrode (not shown), for retrieving and processing cardiac information 300 obtained from the heart of a patient.

Additionally, a therapy device (P) 40 may be permanently or intermittently worn (e.g., implanted or attached) by the patient, such as a heart stimulator, for applying therapeutic signals (e.g., stimulating signals) 400. The therapy device 40 may be an implantable medical device, such as a leadless pacemaker or cardioverter/defibrillator (ICD) device, a cardiac resynchronization therapy (CRT) device, or a therapy device for assisting the heart function, e.g. of a left ventricular assist device (LVAD) pump or an artificial heart.

The heart sensing device or system 30 may be configured to measure an electrogram (EGM) signal as a real-time electrical signal and/or a signal of a bio-impedance type reflecting the intracardiac pressure as a real-time hemodynamic signal. Furthermore, the cardiac information may be an electrocardiogram (ECG) signal and/or a signal representative of the mechanical function of the heart (e.g., a tissue Ultrasound Doppler mode or strain or a mechanical signal reflecting cardiac vibration measured e.g. by an accelerometer or piezoelectric type sensor of the heart sensing device 30, and/or a signal representative of the hemodynamic function of the heart (e.g., an Ultrasound Doppler spectrum of the ventricle).

Furthermore, a user interface (not shown) may be provided for wired or wirelessly inputting and/or outputting therapeutic and/or diagnostic control information from/to a physician or for support in delivery of a therapy and its later optimization.

The controller 130 may be provided for controlling the therapy device 40 based on the real-time cardiac information 30 measured by the heart sensing device 30 of the pacing device worn by the patient.

Additionally, an activity sensor (AS) 10 is provided for measuring an activity level of the patient. The activity sensor 10 may be integrated in the therapy device (if the therapy device 40 is implanted in or attached to the body of the patient) or may be provided as a separate device implanted in or attached to the body of the patient (if the therapy device 40 is not implanted in or attached to the body of the patient).

In the embodiment of Fig. 1, the activity sensor 10 is configured to measure an acceleration in three different directions (e.g., orthogonal directions) and to supply respective acceleration measurement signals A1 to A3 to the controller 20.

As an alternative, the activity sensor 10 may be configured to measure the acceleration in only two or more than three different directions (e.g., which may include combinations of two or three directions).

In an embodiment, the activity sensor 10 may be implemented as an accelerometer (e.g., a microelectromechanical system (MEMS) accelerometer) that outputs an accelerometer signal to determine an oxygen demand of the patient, thus providing information that may be used to implement a truly physiologic rate responsive control.

In embodiments, the activity sensor 10 may be designed to uniquely measure patient's cardiac muscle vibrations generated by the myocardium during cardiac contractions. Such measurements correspond to LV dP/dtₘₐₓ, the gold standard for assessing left ventricular contractility, a key indicator of cardiac performance. It corresponds to the maximal rate of rise of left ventricular pressure (LVP), but it may be determined by myocardial contractility and loading conditions on the ventricle. The value of dP/dtₘₐₓ to represent contractility may be improved by adjusting it to ventricular end-diastolic volume (pre-load) or by calculating dP/dt as a function of LVP during isovolumetric contraction and determining the maximal value.

These real-time measurements are supplied to the controller 20 which uses them to determine pacing settings for the patient. Thereby, the controller 20 can automatically adapt the pacing process to the patient's changing condition.

In embodiments, a rate responsive control algorithm used by the controller 20 may use a relation between variations of amplitude of a first component (EA1) of endocardial acceleration and a heart contractility, which is known to correlate to the inotropic stimulation and in turn to the demand in oxygen by the body. To properly measure EA1 characteristics, a relatively high sampling frequency is required, which may determine an unsuitably high consumption of battery power if the therapy device 40 is a leadless device (e.g., implantable medical device).

According to embodiments, different mode-specific sensing procedures may be implemented (e.g., in the controller 20) to reduce the power consumed by sensor(s) (e.g., the activity sensor 10) and the pacing system/device when used for rate-responsive pacing control.

To achieve this, the controller 10 comprises a mode selecting function 210 for switching between an active mode (AM) and a rest mode (RM) based on a determined activity level of the patient.

In embodiments, reduction of power consumption can be achieved based on the fact that sensor(s) (including the activity sensor 10) for pacing control do not need to be activated with the same frequency and/or in the same way when the patient is determined to be at rest (rest mode selected) and when the patient is active (active mode selected). As there are long periods of rest during day and particularly night, when the patient is sleeping or sitting, substantial reductions of power consumption can be achieved. In an example, during the active mode, the activity sensor 10 may be activated for detecting a signal of interest (e.g., the EA1 component) during a specific and adaptive time window only. Moreover, by configuring the activity sensor 10 to measure the acceleration in two or more directions, but less frequently during a calm period of the cardiac cycle only, the activity level of the patient (e.g., activity starts and stops) can be detected (e.g., in rest mode) without being disturbed by heart sound.

The rest mode is selected when the patient is not physically active. In the rest mode, acquisition (measurement or sampling) of the acceleration is performed in three directions (e.g., three axes), or alternatively two directions (two axes). To reduce consumption, values of the acceleration outputs A1 to A3 in the three directions is only sampled once (or n times, n being small (e.g., n<5)) per cardiac cycle by the controller 20.

Embodiments may be configured so that the sampling frequency does not change between the active mode and the rest mode.

Furthermore, in order to minimize the impact of cardiac acceleration on the recorded signal, the acquisition (measurement or sampling) is performed at a time ("calm" portion) of the cardiac cycle when the cardiac component of the acceleration is small or at least does not vary significantly from one cardiac cycle to the other. This calm portion of the cardiac cycle may be located between the first two components (peaks) of the endocardial acceleration.

The beginning of an activity may be detected based on the acceleration variation between consecutive cardiac cycles. This variation is assumed to be related to body movements, since the signal is sampled at a time (calm period) when the cardiac component of the acceleration is almost constant across cycles. Therefore, when the variation is superior to a predefined threshold (e.g., on the majority (e.g., at least two) of the three axes), a movement is decided. In alternative embodiments, the acceleration variation may be measured in two axes, where the majority may then be two axes.

To avoid over-detections, a persistence criterion may be added by defining the number of cycles out of n consecutive cycles (e.g., n = 10) in which the threshold should be exceeded.

The active mode is selected (activated) once an activity has been detected by the above criteria (algorithm) in rest mode. In the active mode, the acceleration may be measured in one direction (on one axis) only, because the signal of interest (e.g., the EA1 component) is assumed to evolve in the same way in all (two or three) directions (on all (two or three) axes). The appropriate axis may be selected/defined by a calibration at the time of device implantation. Alternatively, a "dynamic" calibration may be done automatically and repeatedly (e.g., periodically).

To reduce energy consumption in the active mode, the acquisition (measurement or sampling) of the acceleration is not performed continuously. An acquisition window is opened after a sensed or paced cardiac event with the aim of recording only the first component (EA1) of the endocardial acceleration.

In order to use the shortest possible window without losing information, the window duration and position relative to the electrical heart signal may be made to evolve during the algorithm operation. The acquisition may for example start with a relatively large window (e.g., 250ms). Depending on the characteristics of the first EA1 signals acquired in this way, once the position of the relevant features of the signal of interest (e.g., EA1 component) within the window is known, the window length can be reduced and its position adapted in order to have the minimum duration still allowing to acquire the signal of interest. Once an optimized window duration and position have been determined, window length and position can keep evolving dynamically, e.g., by tracking the EA1 components (e.g., one positive and one negative peak) based on the history of the position of the signal of interest (e.g., EA1 peaks) in the previous cycles.

However, with a reduced window, even if an effective tracking method is used, it should be considered that for some reason the signal of interest (e.g., one or both of the peaks) may move out of the window, or an isolated phenomenon can lead to an incorrect positioning of the window over many cardiac cycles. To mitigate that risk, a calibration through a large window may be used at the beginning of an acquisition and may be repeated periodically. Another possibility may be to extend the window back to its initial length each time the position (or the amplitude, or both) of the signal of interest (e.g., one of the peaks) changes significantly from one cycle to another, because a "good" peak is suddenly lost and a new peak of the same sign is not in the same place and with the same amplitude as the "lost" one.

Fig. 2 shows a flow diagram of an energy-efficient sensor-based mode switching procedure for rate-responsive pacing according to various embodiments, which may be implemented by the controller 20 of Fig. 1 by executing respective instructions stored in a memory (not shown) of the pacing system.

In step S201 (M-SEL), the mode switching procedure selects the rest mode (RM) or the activity mode (AM) based on a default setting (e.g., rest mode at initial start of the procedure) or a manual setting via a user interface.

When the rest mode is selected in step S201, the procedure branches to step S202B (MAS(CP)) where the above-described multi-axis sensing is performed during the calm period. If an activity is detected in step S203B (AD), as described above, the procedure jumps back to step 5201 and the active mode is selected.

As the active mode has now been selected, the procedure branches to step S202A (SAS(AW)) where the above single-axis sensing procedure with adaptive window is performed.

Several solutions can be considered regarding a switch-back from the active mode to the rest mode (e.g., during recovery). The detection of the end of an activity in step S203A (EoA) can be determined when the criteria for activity detection are no longer met. This may imply to keep collecting "rest mode" samples during the active mode. Alternatively, the end of an activity can be detected in step S203A when the effects of an activity on the signal of interest are no longer visible (e.g., for example when the EA1 peak-to-peak amplitude starts decreasing or reverts to its baseline). In the alternative case, the procedure would respond with a longer delay, but it may be a more physiological way to mimic the cardiac behavior at the end of an activity.

Once the end of the activity has been detected, step S203A may comprise a procedure to decrease the heart rate during recovery, e.g., by using a predefined slope (e.g., the same relation between the heart rate and the signal of interest (e.g., EA1 amplitude) that was used to increase the heart rate during the activity), or by decreasing the cardiac rhythm at a predefined, constant or variable rate. Then, the procedure jumps back to step S201 and the rest mode is selected again.

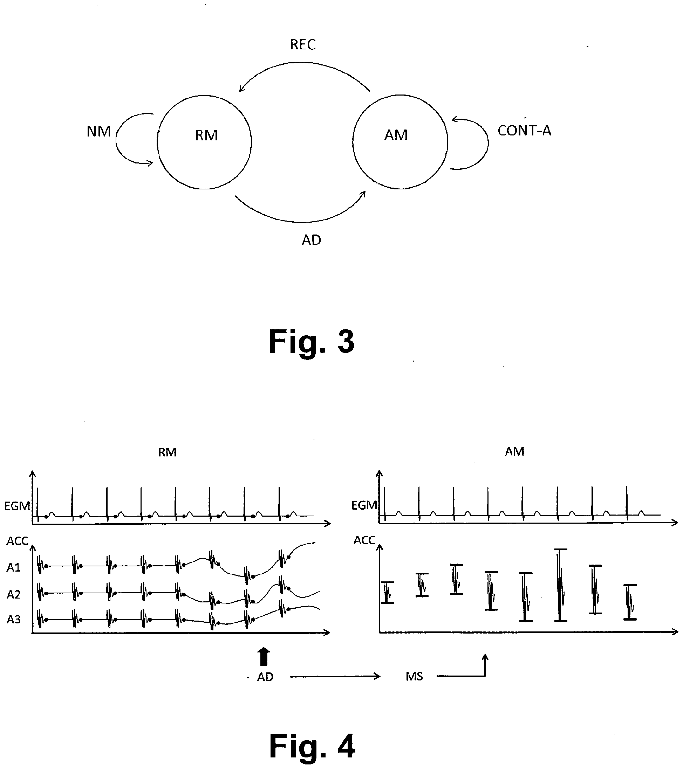

Fig. 3 shows schematically a state diagram of a rate-responsive pacing system according to embodiments.

When the activity-dependent pacing system is in a state of rest mode (RM), it remains in this state as long as no movement (NM) is detected (e.g., activity threshold is not exceeded). When an activity is detected (AD) (e.g., activity threshold exceeded), the system switches to a state of active mode (AM) where is stays as long as a continued activity (CONT-A) is determined (e.g., activity level substantially kept). When the activity level is determined to decrease substantially or to fall below the activity threshold, the system returns/recovers (REC) to the state of rest mode.

Fig. 4 shows respective waveforms of electrograms (EGMs) and sensor signals in different modes of the rate-response pacing system according to an embodiment.

The upper diagrams indicate EGM waveforms (initiating cardiac activity) in the rest mode (left-hand diagram) and in the active mode (right-hand diagram), and the lower diagrams indicate waveforms of endocardiac acceleration (ACC) in three different directions as measured by the activity sensor 10 in the rest mode (left-hand diagram with three waveforms A1 to A3 in different directions) and in the activity mode (right-hand diagram with one waveform in one direction).

As can be gathered from the left-hand waveforms, measurement times (dark spots) are located in calm portions with low cardiac activity as indicated by the EGM signal. In the last two measurements of the lower left-hand diagram, the measured acceleration increases until it meets the threshold criterion/criteria and an activity is detected (AD). In response, the system switches to the activity mode (mode switch (MS)) of the right-hand diagrams where an increased acceleration is measured in a single direction and during adaptive windows only.

To summarize, a medical device and a method for controlling a heart rate of a patient have been described, wherein a sensor is used for detecting an acceleration of the medical device in two or more different directions and a controller is configured to activate the sensor with different timing in a rest mode and in an active mode to sample the detected acceleration. In the rest mode, the detected acceleration is sampled in the two or more different directions a predetermined number of times per cardiac cycle during a calm period of the cardiac cycle with low signal amplitude, and in the active mode, the detected acceleration is sampled per one or more cardiac cycles in one direction only and during a specific adaptive window that includes a signal of interest, e.g., a systolic component of an endocardial acceleration of the cardiac cycle.

While the invention has been illustrated and described in detail in the drawings and foregoing description, such illustration and description are to be considered illustrative or exemplary and not restrictive. The invention is not limited to the disclosed embodiments. The proposed energy-efficient rate-responsive pacing can be used for any medical devices for treatment of human beings and animals. In particular, the invention can be applied to any kind of pacing devices or defibrillators to reduce their energy consumption. Moreover, any kind sensor which is suitable for detecting an acceleration can be used.

Other variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. The foregoing description details certain embodiments of the invention. It will be appreciated, however, that no matter how detailed the foregoing appears in the text, the invention may be practiced in many ways, and is therefore not limited to the embodiments disclosed. It should be noted that the use of particular terminology when describing certain features or aspects of the invention should not be taken to imply that the terminology is being re-defined herein to be restricted to include any specific characteristics of the features or aspects of the invention with which that terminology is associated.

The described operations or procedures like those indicated in Fig. 2 can be implemented as program code means of a computer program and/or as dedicated hardware of the receiver devices or transceiver devices, respectively. The computer program may be stored and/or distributed on a suitable medium, such as an optical storage medium or a solid-state medium, supplied together with or as part of other hardware, but may also be distributed in other forms, such as via the Internet or other wired or wireless telecommunication systems.

## Claims

1. A medical device for controlling a heart rate of a patient, comprising:'
a sensor for detecting an acceleration of the medical device in two or more different directions;
a controller for activating the sensor with different timing in a rest mode and in an active mode to sample the detected acceleration;
wherein the controller is configured to:
- in the rest mode, sample the detected acceleration in the two or more different directions a predetermined number of times per cardiac cycle during a calm period of the cardiac cycle with low cardiac signal amplitude; and
- in the active mode, sample the detected acceleration per one or more cardiac cycles in one direction only and during a specific adaptive window that includes a signal of interest, in particular a systolic component of an endocardial acceleration of the cardiac cycle.

2. The device of claim 1, wherein the medical device is an implantable medical device, in particular a leadless pacemaker.

3. The device of claim 1 or 2, wherein the controller is configured to sample the detected acceleration in three different directions in the rest mode.

4. The device of any of the preceding claims, wherein the controller is configured to sample the detected acceleration at least once per one or more cardiac cycles in the rest mode.

5. The device of any of the preceding claims, wherein the controller is configured to detect a movement of the patient when a variation of a sampled value of the detected acceleration exceeds a predefined threshold on at least one of the two or more different directions in the rest mode.

6. The device of any of the preceding claims, wherein the controller is configured to detect a movement of the patient when a predefined threshold is exceeded on at least one of the two or more different directions.

7. The device of any of the preceding claims, wherein the controller is configured to use an enlarged window for intermediate calibration of the window position at the beginning of the active mode and/or periodically during an acceleration measurement in the active mode.

8. The device of any one of the preceding claims, wherein the controller is configured to use the rest mode and the active mode for rate responsive pacing.

9. The device of any one of the preceding claims, wherein the device is configured to control the heart rate of the patient in the active mode by following a predetermined relation between an amplitude of the endocardial acceleration amplitude and a pacing rate.

10. The device of any one of the preceding claims, wherein the controller is configured to switch from the active mode to the rest mode when no movement of the patient is detected for a predetermined number of samples and/or when the endocardial acceleration has decreased to a predetermined level.

11. The device of any one of the preceding claims, wherein the controller is configured to decrease the pacing rate of the patient after a switch from the active mode to the rest mode by using a predetermined slope.

12. A method of controlling a medical device that controls a heart rate of a patient, the method comprising:
detecting an acceleration of the medical device in two or more different directions;
providing a rest mode in which the detected acceleration is sampled in the two or more different directions a predetermined number of times per one or more cardiac cycles during a calm period of the cardiac cycle with low signal amplitude; and
providing an active mode in which the detected acceleration is sampled per one or more cardiac cycles in one direction only and during a specific adaptive window that includes a signal of interest, in particular a systolic component of an endocardial acceleration of the cardiac cycle.

13. The method of claim 12, further comprising controlling the heart rate of the patient in the active mode by following a predetermined relation between an amplitude of the endocardial acceleration and the heart rate.

14. The method of claim 12 or 13, further comprising switching from the active mode to the rest mode when no movement of the patient is detected for a predetermined number of samples and/or when the endocardial acceleration has decreased to a predetermined level.

15. A computer program product comprising code means for generating the steps of any one of claims 12 to 14 when run on a controlling device.
